(19) Europäisches Patentamt
European Patent Office
Office européen des brevets

(11) Veröffentlichungsnummer: **0 121 651**
B1

(12) # EUROPÄISCHE PATENTSCHRIFT

(45) Veröffentlichungstag der Patentschrift: **01.07.87**

(51) Int. Cl.[4]: **C 07 C 143/833**, C 07 C 157/12, A 01 N 47/34

(21) Anmeldenummer: **84100402.1**

(22) Anmeldetag: **16.01.84**

(54) **Phenyl-sulfonylharnstoffe als Bakterizide.**

(30) Priorität: **17.01.83 US 458587**
**21.12.83 US 563841**

(43) Veröffentlichungstag der Anmeldung: **17.10.84 Patentblatt 84/42**

(45) Bekanntmachung des Hinweises auf die Patenterteilung: **01.07.87 Patentblatt 87/27**

(84) Benannte Vertragsstaaten: **AT BE CH DE FR IT LI LU NL SE**

(56) Entgegenhaltungen: **EP-A-0 044 807**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**
Erfinder: **Töpfl, Werner, Dr., Dorneckstrasse 68, CH- 4143 Dornach (CH)**
Erfinder: **Kristinsson, Haukur, Dr., Kreuzackerweg 19, CH- 4103 Bottmingen (CH)**
Erfinder: **Eckhardt, Wolfgang, Dr., Breslauerstrasse 14, D-7850 Lörrach (DE)**

(74) Vertreter: **Zumstein, Fritz, Dr., Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1987

**Beschreibung**

Die vorliegende Erfindung betrifft neue N-[2-(1,2-Dichlorvinyloxy)-phenylsulfonyl]-(thio)harnstoffe der nachstehenden Formel I. Sie betrifft ferner die Herstellung dieser Substanzen sowie agrochemische Mittel, die als Wirkstoff mindestens eine dieser Verbindungen enthalten. Die Erfindung betrifft auch die Verwendung der Wirkstoffe oder der Mittel zur Bekämpfung von schädlichen Bakterien, insbesondere phytopathogener Arten.

Bei den erfindungsgemässen Verbindungen handelt es sich um solche der allgemeinen Formel I

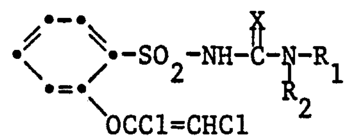

(I)

worin

X für Sauerstoff oder Schwefel steht;

$R_1$ Wasserstoff, $C_1$-$C_{12}$-Alkyl, $C_2$-$C_6$-Alkenyl oder $C_2$-$C_6$-Alkinyl bedeutet; und

$R_2$ für Wasserstoff, $C_1$-$C_{12}$-Alkyl, $C_2$-$C_6$-Alkenyl, $C_2$-$C_6$-Alkinyl oder $C_1$-$C_6$-Alkoxy steht;

sowie die Salze dieser Verbindungen.

Im Umfang der Formel I sind die Harnstoffe gegenüber den Thioharnstoffen bevorzugt.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Äthylamin, Propylamin, i-Propylamin, die vier isomerem Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin. Ferner sind tricyclische Stickstoffbasen, wie z. B. 1,4-Diazabicyclo[2,2,2]octan, 1,8-Diazabicyclo[5.4.0]undec-7-en oder 1,5-Diazabicyclo[4.3.0]-non-5-en geeignet.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter dem Begriff Alkyl selbst oder als Bestandteil eines anderen Substituenten, wie Alkoxy etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl, Nonyl, Decyl, Undecyl, Dodecyl usw. sowie ihre Isomeren, wie z. B. Isopropyl, Isobutyl, tert.-Butyl, Isopentyl usw.. Alkenyl steht z. B. für Propenyl-(1), Allyl, Butenyl-(1), Butenyl-(2) oder Butenyl-(3) sowie Ketten mit mehreren Doppelbindungen. Alkinyl bedeutet z. B. Propinyl-(2), Propargyl, Butinyl-(1), Butinyl-(2) usw., vorzugsweise Propargyl.

Die Verbindungen der Formel I sind bei Raumtemperatur Öle, Harze oder überwiegend kristalline Feststoffe, die sich durch sehr wertvolle bakterizide Eigenschaften auszeichnen. Sie lassen sich beispielsweise auf dem Agrarsektor oder verwandten Gebieten präventiv und kurativ zur Bekämpfung phytopathogener Bakterien, insbesondere Xanthomonas-Arten einsetzen. Die erfindungsgemässen Wirkstoffe der Formel I zeichnen sich in weiten Anwendungskonzentrationen durch eine hohe bakterizide Aktivität und grosse Wirkungsbreite aus und können problemlos insbesondere im Agrarbereich eingesetzt werden.

Folgende Wirkstoffgruppen sind auf Grund ihrer ausgeprägten bakteriziden insbesondere phytobakteriziden Aktivität bevorzugt:

Gruppe Ia: Verbindungen der Formel I, worin X für Sauerstoff oder Schwefel steht; $R_1$ für Wasserstoff oder $C_1$-$C_6$-Alkyl steht; und $R_2$ Wasserstoff, $C_1$-$C_6$-Alkyl, $C_2$-$C_6$-Alkenyl, $C_2$-$C_6$ -Alkinyl oder $C_1$-$C_6$-Alkoxy bedeutet.

Gruppe Ib: Verbindungen der Formel I, worin X für Sauerstoff oder Schwefel steht; $R_1$ für Wasserstoff oder $C_1$-$C_3$-Alkyl steht; und $R_2$ $C_1$-$C_6$-Alkyl oder $C_1$-$C_6$-Alkoxy bedeutet.

Gruppe Ic: Verbindungen der Formel I, worin X für Sauerstoff steht; $R_1$ für Wasserstoff oder $C_1$-$C_3$-Alkyl steht; und $R_2$ $C_1$-$C_6$-Alkyl bedeutet.

Interessante Untergruppen sind ferner:

Gruppe Id: Verbindungen der Formel I, worin X für Sauerstoff oder Schwefel steht; $R_1$ für Wasserstoff oder $C_1$-$C_3$-Alkyl steht; und $R_2$ $C_2$-$C_6$-Alkenyl oder $C_2$-$C_6$-Alkinyl bedeutet.

Gruppe Ie: Verbindungen der Formel I, worin X für Sauerstoff steht; $R_1$ für Wasserstoff oder $C_1$-$C_3$-Alkyl steht; und $R_2$ $C_2$ -$C_4$-Alkenyl oder $C_2$-$C_4$-Alkinyl bedeutet.

Gruppe If: Verbindungen der Formel I, worin X für Sauerstoff steht und $R_1$ und $R_2$ unabhängig voneinander für Wasserstoff, $C_2$-$C_6$-Alkenyl oder $C_2$-$C_6$-Alkinyl bedeuten.

Besonders bevorzugte Einzelsubstanzen sind z. B.:
N-[2-(1,2-Dichlorvinyloxy)phenylsulfonyl]-harnstoff (Verb. Nr. 1.1);
N-[2-(1,2-Dichlorvinyloxy)phenylsulfonyl]-N'-methylharnstoff (Verb. Nr. 1.2);
N-[2-(1,2-Dichlorvinyloxy)phenylsulfonyl]-N'-isopropylharnstoff (Verb. Nr. 1.5);
N-[2-(1,2-Dichlorvinyloxy)phenylsulfonyl]-N'-n-butylharnstoff (Verb. Nr. 1.6);
N-[2-(1,2-Dichlorvinyloxy)phenylsulfonyl]-N'-methoxy-N'-methylharnstoff (Verb. Nr. 1.29);
N-[2-(1,2-Dichlorvinyloxy)phenylsulfonyl]-N'-allylharnstoff (Verb. Nr. 1.20);
N-[2-(1,2-Dichlorvinyloxy)phenylsulfonyl]-N',N'-dimethylharnstoff (Verb. Nr. 1.24);
N-[2-(1,2-Dichlorvinyloxy)phenylsulfonyl]-N'-methyl-N'-ethylharnstoff (Verb. Nr. 1.25);
N-[2-(1,2-Dichlorvinyloxy)pheylsulfonyl]-N'-methyl-N'-allylharnstoff (Verb. Nr. 1.32);
N-[2-(1,2-Dichlorvinyloxy)phenylsulfonyl]-N'-methylthioharnstoff (Verb. Nr. 2.2);
N-[2-(1,2-Dichlorvinyloxy)phenylsulfonyl]-N'-isopropylthioharnstoff (Verb. Nr. 2.5);
N-[2-(1,2-Dichlorvinyloxy)phenylsulfonyl]-N'-allylthioharnstoff Verb.Nr. 2.20).

Die erfindungsgemässe Herstellung der Verbindungen der Formel I erfolgt in einem reaktionsinerten, organischen Lösungsmittel, indem man

a) ein 2-(1,2-Dichlorvinyloxy)phenylsulfonyliso(thio)cyanat der Formel II

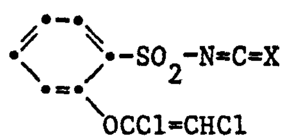

(II),

vorzugsweise in Anwesenheit einer Base, mit einem Amin der Formel III

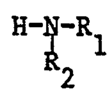

(III)

umsetzt, oder

b) das 2-(1,2-Dichlorvinyloxy)phenylsulfonamid der Formel IV

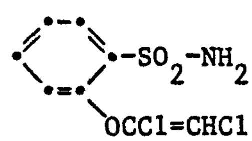

(IV),

in Gegenwart einer Base, mit einem Carbamoylhalogenid der Formel

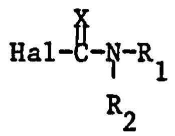 (V)

umsetzt; oder

c) zur Herstellung von Verbindungen der Formel I, worin $R_1$ für Wasserstoff steht, indem man das 2-(1,2-Dichlorvinyloxy)phenylsulfonamid der obigen Formel IV, in Gegenwart einer Base, mit einem Iso(thio)cyanat der Formel VI

$X = C = N - R_2$ (VI)

umsetzt; wobei die Substituenten $R_1$, $R_2$ und X die unter Formel I angegebenen Bedeutungen haben und Hal für Halogen, vorzugsweise Chlor oder Brom, insbesondere Chlor steht.

Die erhaltenen Harnstoffe der Formel I können gewünschtenfalls mittels Aminen, Alkalimetall- oder Erdalkalimetallhydroxiden oder quaternären Ammoniumbasen in Salze übergeführt werden. Dieses geschieht beispielsweise durch Umsetzen mit der äquimolaren Menge Base und Verdampfen des Lösungsmittels.

Die Verbindungen der Formeln III und VI sind allgemein bekannt oder werden nach an sich bekannten Methoden hergestellt.

Die Verbindungen der Formeln II und IV bzw. deren Herstellung wird in der Europäischen Offenlegungsschrift EP 44 807 beschrieben.

Diese Umsetzungen a, b und c zu Verbindungen der Formel I werden vorteilhafterweise in aprotischen, inerten, organischen Lösungsmitteln oder Lösungsmittelgemischen vorgenommen. Als Lösungs- oder Verdünnungsmittel kommen beispielsweise in Frage: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Ethylenglykoldimethylether, Diethylenglykoldimethylether, Anisol, Dioxan, Tetrahydrofuran; Nitrile wie Acetonitril, Propionitril; z.T. N,N-alkylierte Amide wie Dimethylformamid; Dimethylsulfoxid; Ketone wie Aceton, Diethylketon, Methylethylketon und Gemische solcher Lösungsmittel untereinander. In manchen Fällen kann das Reagens selbst als Lösungsmittel dienen.

Die Reaktionstemperaturen liegen im allgemeinen zwischen -20° und +120°C, vorzugsweise zwischen 0° und +30°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches erhitzt. Die Reaktionszeiten können ebenfalls durch Zugabe einer Tropfen Base als Reaktionskatalysator verkürzt werden.

Als Basen können sowohl organische Basen wie Amine, z. B. tertiäre Amine (wie Triethylamin, Trimethylamin, Tripropylamin, N-Methylpiperidin, 1,4-Diazabicyclo(2,2,2)octan 1,8-Diazabicyclo[5.4.0]undec-7-en und ähnliche), Pyridin und Pyridinbasen (wie 4-Dimethylamino-pyridin, 4-Pyrrolidylaminopyridin, Picoline, Lutidin usw.), als auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden. Bei Variante a kann die Verbindung der Formel III selbst als Base dienen.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Ether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Massnahmen.

Es wurde überraschend festgestellt, dass Verbindungen der Formel I ein für praktische Bedürfnisse sehr günstiges Bakterizid-Spektrum gegen phytopathogene Bakterien aufweisen. Sie besitzen sehr vorteilhafte kurative z.T. systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mirkroorganismen eingedämmt oder vernichtet werden, wobei auch später zuwachsende Pflanzenteile von derartigen Schädlingen verschont bleiben.

Die Wirkstoffe der Formel I und die daraus hergestellten Mittel sind insbesondere gegen Bakterien der Ordnung Pseudomonadales, so z. B. gegen Bakterien der Familie der Pseudomonadaceae, wie Pseudomonas- und Xanthomonas-Arten (Pseudomonas tomato, Pseudomonas tabaci, Pseudomonas morsprunorum,

Pseudomonas phaseolicola, Pseudomonas lachrymans, Xanthomonas campestris, Xanthomonas oryzae, Xanthomonas vesicatoria) wirksam.

Die Erfindung betrifft somit auch bakterizide Mittel sowie die Verwendung der Verbindungen der Formel I zur Bekämpfung phytopathogener Bakterien, insbesondere pflanzenschädigender Xanthomonas-Arten sowie die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat. Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen), vorzugsweise Reis und Paprika.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochmeischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte,

applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z. B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Lunge, Leber, Eidotter oder Sojabohnen gewinnen kann. Verwendbare Handelsmischungen sind z. B. Phosphatidylcholin-Mischungen. Synthetische Phospholipide sind z. B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen rest mit 8 bis 22 C-Atomen auf, wobei Alkyl auch den teil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Ethylenoxid-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykoläthergruppen und 10 bis 100 g Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" BC Publishing Corp., Ridgewood New Jersey, 1981
Helmut Stache "Tensid-Taschenbuch" Carl Hanser-Verlag München/Wien 1981.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige argrochemische Mittel sind ein Bestandteil der vorliegenden Erfindung.

Die nachfolgenden Beispiele dienen zur näheren Erläuterung der Erfindung, ohne dieselbe einzuschränken.

**Herstellungsbeispiele:**

**Beispiel H1**: Herstellung von

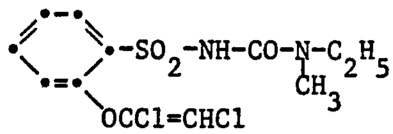

(Verb. Nr. 1.25)

N-[2-(1,2-Dichlorvinyloxy)phenylsulfonyl]-N'-methyl-N'-ethylharnstoff

Zu einer Lösung von 10,3 g 2-(1,2-Dichlorvinyloxy)phenyl-isocyanat in 100 ml absolutem Dioxan wird eine Lösung von 2,1 g N-Methylethylamin in 50 ml absolutem Dioxan bei 20 bis 25° C zugetropft und das Gemisch 15 Stunden bei Raumtemperatur weitergerührt. Anschliessend wird die Reaktionsmischung eingedampft und der Rückstand durch Versetzen mit einem Gemisch von Aceton/Hexan zur Kristallisation gebracht. Das Produkt wird abfiltriert und getrocknet. Ausbeute 7,5 g. Smp. 179-180° C.

**Beispiel H2**: Herstellung von

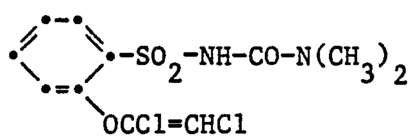

(Verb. Nr. 1.24)

N-[2-(1,2-Dichlorvinyloxy)phenylsulfonyl]-N',N'-dimethylharnstoff

Eine Mischung aus 10,7 g 2-(1,2-Dichlorvinyloxy)phenylsulfonamid, 12,2 g 1,8-Diazabicyclo[5.4.0]undec-7-en (1,5-5) in 100 ml Dichlormethan wird bei 20 bis 25°C tropfenweise mit einer Lösung von 10,2 g Dimethylcarbamoylchlorid in 10 ml Dichlormethan versetzt und das Gemisch 2 Stunden unter Rückfluss erhitzt. Anschliessend wird das Lösungsmittel abgedampft und der Rückstand in 5 %-iger Sodalösung gelöst. Die Lösung wird filtriert und mit 10 %-iger Salzsäure angesäuert, wobei das Produkt kristallin ausfällt. Nach dem Filtrieren und Trocknen erhält man 12,1 g N-[2-(1,2-Dichlorvinyloxy)-Phenylsulfonyl]-N',N'-dimethylharnstoff. Smp. 218-220° C.

**Beispiel H3**: Herstellung von

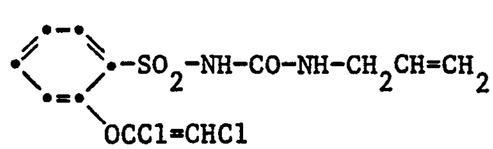

(Verb. Nr. 1.20)

N-[2-(1,2-Dichlorvinyloxy)phenylsulfonyl]-N'-allylharnstoff

Eine Mischung aus 10,7 g 2-(1,2-Dichlorvinyloxy)phenylsulfonamid und 6,1 g 1,8-Diazabicyclo[5.4.0]undec-7-en(1,5-5) in 100 ml Dichlormethan wird tropfenweise und unter Rühren mit einer Lösung von 3,3 g Allylisocyanat in 10 ml Dichlormethan versetzt. Nach Abklingen der exothermen Reaktion wird das Reaktionsgemisch noch 1 Stunde bei 20° bis 25°C weiter gerührt und dann das Lösungsmittel abgedampft. Der Rückstand wird in 5%-iger Sodalösung gelöst. Die Lösung wird filtriert und mit 10%-iger Salzsäure angesäuert, wobei das Produkt kristallin ausfällt. Nach dem Filtrieren und Trocknen erhält man 13,2 g N-[2-(1,2-Dichlorvinyloxy)phenylsulfonyl]-N'-allylharnstoff. Smp. 187-188°C.

**Beispiel H4**: Herstellung von

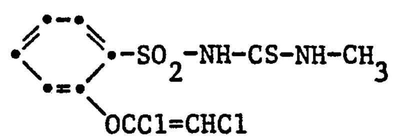

(Verb. Nr. 2.2)

N-[2-(1,2-Dichlorvinyloxy)phenylsulfonyl]-N'-methylthioharnstoff

13,4 g 2-(1,2-Dichlorvinyloxy)phenylsulfonamid und 3,65 g Methylisothiocyanat werden in 50 ml Acetonitril gelöst und unter Kühlen (0° bis 5°C) und Rühren mit 7,8 g 1,8-Diazabicyclo[5.4.0]undec-7-en (1,5-5) versetzt. Die resultierende rote Reaktionslösung wird 24 Stunden stehen gelassen, dann mit 4,8 g Methansulfonsäure versetzt und mit Wasser auf ein Volumen von 1 Liter verdünnt. Die abgeschiedenen Kristalle werden abfiltriert, mit Methanol gewaschen und getrocknet. Ausbeute 15 g. Smp. 185-187° (Zers.).

**Beispiel H5**: Herstellung von

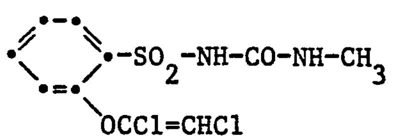

(Verb. Nr. 1.2)

N-[2-(1,2-Dichlorvinyloxy)phenylsulfonyl]-N'-methylharnstoff

Eine Suspension von 6,7 g 2-(1,2-Dichlorvinyloxy)phenylsulfonamid in 50 ml Dichlormethan wird mit 1,9 g Methylisocyanat versetzt. Zu dieser Mischung werden bei 20 bis 25°C im Verlauf von 10 Minuten 3,3 g Triethylamin zugetropft, wobei eine klare Lösung entsteht. Diese wird noch eine Stunde weitergerührt und anschliessend bis zur Trockene eingedampft. Der Rückstand wird in 5 %-iger wässriger Sodalösung gelöst und mit 10%-iger Salzsäure angesäuert, wobei das Produkt kristallin ausfällt. Nach dem Filtrieren und Trocknen erhält man 7,8 g N-[2-(1,2-Dichlorvinyloxy)phenylsulfonyl]-N'-methylharnstoff. Smp. 228-229°C.

Analog zu den beschriebenen Arbeitsweisen werden auch die nachfolgend aufgeführten Verbindungen der Formel I hergestellt:

**Tabelle 1**: Verbindungen der Formel

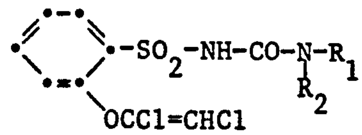

| Verb. Nr. | $R_1$ | $R_2$ | physik.Konst.[°C] |
|---|---|---|---|
| 1.1 | H | H | Smp. 219-221° |
| 1.2 | H | $CH_3$ | Smp. 228-229° |
| 1.3 | H | $C_2H_5$ | |
| 1.4 | H | $C_3H_7$-n | |
| 1.5 | H | $C_3H_7$-i | Smp. 156-160° |
| 1.6 | H | $C_4H_9$-n | Smp. 123-126° |
| 1.7 | H | $C_4H_9$-s | |
| 1.8 | H | $C_4H_9$-t | |
| 1.9 | H | $C_5H_{11}$-n | |
| 1.10 | H | $C_2H_4C(CH_3)HCH_3$ | |
| 1.11 | H | $C_6H_{13}$-n | |
| 1.12 | H | $C_2H_4C(CH_3)_3$ | |
| 1.13 | H | $OCH_3$ | Smp.154-156° |
| 1.14 | H | $OC_2H_5$ | |
| 1.15 | H | $OC_3H_7$-i | |
| 1.16 | H | $OC_3H_7$-n | |
| 1.17 | H | $OC_4H_9$-n | |
| 1.18 | H | $OC_5H_{11}$-n | |
| 1.19 | H | $OC_6H_{13}$-n | |
| 1.20 | H | $CH_2CH=CH_2$ | Smp.187-188° |
| 1.21 | H | $CH_2C\equiv CH$ | |
| 1.22 | H | $C(CH_3)_2C\equiv CH$ | Smp. 153-156° |
| 1.23 | H | $CH_2CH_2CH=CH_2$ | |
| 1.24 | $CH_3$ | $CH_3$ | Smp. 218-220° |
| 1.25 | $CH_3$ | $C_2H_5$ | Smp.179-180° |
| 1.26 | $CH_3$ | $C_3H_7$-n | |
| 1.27 | $CH_3$ | $C_3H_7$-i | |
| 1.28 | $C_2H_5$ | $C_2H_5$ | |
| 1.29 | $CH_3$ | $OCH_3$ | Smp. 154-156° |
| 1.30 | $CH_3$ | $OC_2H_5$ | |
| 1.31 | $CH_3$ | $OC_3H_7$-n | |
| 1.32 | $CH_3$ | $CH_2CH=CH_2$ | Smp.144-146° |
| 1.33 | $CH_3$ | $CH_2CH_2CH=CH_2$ | |
| 1.34 | $CH_2CH=CH_2$ | $CH_2CH=CH_2$ | |
| 1.35 | $C_2H_5$ | $CH_2CH=CH_2$ | |
| 1.36 | $C_3H_7$-n | $CH_2CH=CH_2$ | Harz |
| 1.37 | $C_2H_5$ | $CH_2C(CH_3)=CH_2$ | |
| 1.38 | $C_3H_7$-i | $CH_2C(CH_3)=CH_2$ | Harz |
| 1.39 | $CH_3$ | $CH_2C(CH_3)=CH_2$ | |
| 1.40 | H | $CH_2C(CH_3)=CH_2$ | |
| 1.41 | $CH_3$ | $CH_2C\equiv CH$ | semikristallin |
| 1.42 | $CH_3$ | $C(CH_3)_2C\equiv CH$ | |

**Tabelle 2**: Verbindungen der Formel

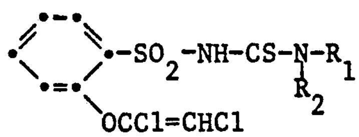

| Verb. Nr. | $R_1$ | $R_2$ | physik. Konst. [°C] |
|---|---|---|---|
| 2.1 | H | H | |
| 2.2 | H | $CH_3$ | Smp.185-187° |
| 2.3 | H | $C_2H_5$ | |
| 2.4 | H | $C_3H_7$-n | |
| 2.5 | H | $C_3H_7$-i | Smp.146-148° |
| 2.6 | H | $C_4H_9$-n | |
| 2.7 | H | $C_4H_9$-s | |
| 2.8 | H | $C_4H_9$-t | |
| 2.9 | H | $C_5H_{11}$-n | |
| 2.10 | H | $C_2H_4C(CH_3)HCH_3$ | |
| 2.11 | H | $C_6H_{13}$-n | |
| 2.12 | H | $C_2H_4C(CH_3)_3$ | |
| 2.13 | H | $OCH_3$ | zähes Öl |
| 2.14 | H | $OC_2H_5$ | semikristallin |
| 2.15 | H | $OC_3H_7$-i | |
| 2.16 | H | $OC_3H_7$-n | |
| 2.17 | H | $OC_4H_9$-n | |
| 2.18 | H | $OC_5H_{11}$-n | |
| 2.19 | H | $OC_6H_{13}$-n | |
| 2.20 | H | $CH_2CH=CH_2$ | Smp. 129-131° |
| 2.21 | H | $CH_2C\equiv CH$ | |
| 2.22 | H | $C(CH_3)_2C\equiv CH$ | |
| 2.23 | H | $CH_2CH_2CH=CH_2$ | |
| 2.24 | $CH_3$ | $CH_3$ | Harz |
| 2.25 | $CH_3$ | $C_2H_5$ | |
| 2.26 | $CH_3$ | $C_3H_7$-n | |
| 2.27 | $CH_3$ | $C_3H_7$-i | Harz |
| 2.28 | $C_2H_5$ | $C_2H_5$ | |
| 2.29 | $CH_3$ | $OCH_3$ | |
| 2.30 | $CH_3$ | $OC_2H_5$ | |
| 2.31 | $CH_3$ | $OC_3H_7$-n | |
| 2.32 | $CH_3$ | $CH_2CH=CH_2$ | |
| 2.33 | $CH_3$ | $CH_2CH_2CH=CH_2$ | |
| 2.34 | $CH_2CH=CH^2$ | $CH_2CH=CH_2$ | |
| 2.35 | $C_2H_5$ | $CH_2CH=CH_2$ | |
| 2.36 | $C_3H_7$-n | $CH_2CH=CH_2$ | |
| 2.37 | $C_2H_5$ | $CH_2C(CH_3)=CH_2$ | |
| 2.38 | $C_3H_7$-i | $CH_2C(CH_3)=CH_2$ | |
| 2.39 | $CH_3$ | $CH_2C(CH_3)=CH_2$ | |
| 2.40 | H | $CH_2C(CH_3)=CH_2$ | |
| 2.41 | $CH_3$ | $CH_2C\equiv CH$ | |
| 2.42 | $CH_3$ | $C(CH_3)_2C\equiv CH$ | |

**Formulierungsbeispiele für flüssige Wirkstoffe der Formel I (% = Gewichtsprozent)**

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenol-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethylether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190° C) | - | - | 94% | - |
| (MG = Molekulargewicht) | | | | |

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschließend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

**Formulierungsbeispiele für feste Wirkstoffe der Formel 1 (% = Gewichtsprozent)**

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na.Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10% |
| Octylphenolpolyethylenglykolether (4-5 Mol Ethylenoxid) | 3% |
| Ca-Dodecylbenzolsulfonat | 3% |
| Ricinusälpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit dem Träger vermischt auf einer geeigneten Mühle vermahlen wird.

F8. Extruder Granulat

| | |
|---|---|
| Wirkstoff aus den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40% |
| Ethylenglykol | 10% |
| Nonylphenolpolyethylenglykolether (15 Mol Ethylenoxid) | 6% |
| NaLigninsulfonat | 10% |
| Carboxymethylcellulose | 1% |
| 37%ige wässrige Formaldehyd-Lösung | 0,2% |
| Silikonöl in Form einer 65%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele:**

**Beispiel B1**: Wirkung gegen Xanthomonas oryzae auf Reis

a) Residual-protektive Wirkung

Reispflanzen der Sorte "Caloro" oder "S6" wurden nach 3-wöchiger Anzucht im Gewächshaus mit der Prüfsubstanz in Form einer Spritzbrühe (0,06 % Aktivsubstanz) besprüht. Nach eintägigem Antrocknen dieses Spritzbelages wurden die Pflanzen in einem Klimaraum bei 24°C und 75-85 % relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der residualen Wirksamkeit der Prüfsubstanz.

b) Systemische Wirkung

Reispflanzen der Sorte "Caloro" oder "S6" wurden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung wurden die Pflanzen in einem Klimaraum bei 24°C und 75-85 % relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der systemischen Wirksamkeit der Prüfsubstanz.

Verbindungen aus den Tabellen zeigten eine gute Wirkung gegen Xanthomonas-Bakterien, so hemmten bei der residual-protektiven und systemischen Behandlung von Reis unter anderen die Verbindungen Nr. 1.1, 1.2, 1.5, 1.6, 1.13, 1.20, 1.22, 1.24, 1.25, 1.32, 2.2, 1.29 2.5 und 2.20 das Auftreten von nekrotischen Flecken fast vollständig (0 bis 5 %). Die Verbindungen 1.2 und 1.25 verhinderten auch bei noch niedrigeren Aufwandmengen den Pilzbefall vollständig (0 %). Unbehandelte aber infizierte Reispflanzen (Kontrolle) wiesen eine Nekrose von 100 % auf.

**Beispiel B2**: Wirkung gegen Xanthomonas vesicatoria auf Paprika

a) Residual-protektive Wirkung

Paprikapflanzen der Sorte "California Wonder" wurden nach 3-wöchiger Anzucht im Gewächshaus mit der Prüfsubstanz in Form einer Spritzbrühe (0,06 % Aktivsubstanz) besprüht. Nach eintägigem Antrocknen dieses Spritzbelages wurden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nektrotische, aufgehellte Flecken. Das Ausmass dieser Flecken diente zur Beurteilung der residualen Wirksamkeit der Prüfsubstanz.

b) Systemische Wirkung

Paprikapflanzen der Sorte "California Wonder" wurden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung wurden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der systemischen Wirksamkeit der Prüfsubstanz. Verbindungen aus beiden Tabellen verhinderten das Auftreten von Flecken fast vollständig.

In beiden Versuchen hemmten die in Beispiel B1 genannten Substanzen das Auftreten von Nekrosen beinahe vollständig (0 bis 5 %). Kontrolle 100 %.

**Beispiel B3**: Wirkung gegen Pseudomonas tomato auf Tomaten

a) Residual-protektive Wirkung

Tomatenpflanzen der Sorte Rentita wurden nach 3-wöchiger Anzucht im Gewächshaus mit der Prüfsubstanz in Form einer Spritzbrühe (0,06 % Aktivsubstanz) besprüht. Nach eintägigem Antrocknen dieses Spritzbelages wurden die Pflanzen in einem Klimaraum bei 22°C und 95-100 % relativer Luftfeuchtigkeit aufgestellt und durch. Besprühen der Blattunterseiten mit einer standardisierten Suspension von Pseudomonas tomato infiziert. Nach 8-tägiger Inkubation in demselben Raum entstanden auf den Blättern kleine, schwarze Flecken mit gelbem Hof. Die durchschnittliche Anzahl der Flecken pro Blatt diente als Bewertungsgrundlage für die Wirksamkeit der Prüfsubstanz.

b) Systemische Wirkung
Tomatenpflanzen der Sorte Rentita wurden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung wurden die Pflanzen in einem Klimaraum bei 22°C und 95-100 % relativer Luftfeuchtigkeit aufgestellt und durch Besprühen der Blattunterseiten mit einer standardisierten Suspension von Pseudomonas tomato infiziert. Nach 8-tägiger Inkubation in demselben Raum entstanden auf den Blättern kleine, schwarze Flecken mit gelbem Hof. Die durchschnittliche Anzahl solcher Flecken pro Blatt diente als Bewertungsgrundlage für die Wirksamkeit der Prüfsubstanz.

In beiden Tests hemmten die in Beispiel B1 genannten Substanzen das Auftreten von Blattnekrosen fast vollständig (0 bis 5 %). Kontrolle 100 %.

**Patentansprüche**

für die Vertragsstaaten: BE, CH, LI, DE, FR, IT, LU, NL, SE.

1. Verbindungen der Formel I,

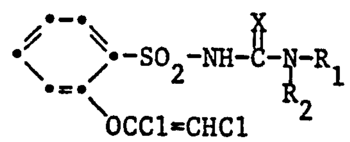

(I)

worin
X für Sauerstoff oder Schwefel steht;
$R_1$ Wasserstoff $C_1$-$C_{12}$-Alkyl, $C_2$-$C_6$-Alkenyl oder $C_2$-$C_6$-Alkinyl bedeutet; und
$R_2$ für Wasserstoff, $C_1$-$C_{12}$-Alkyl, $C_2$-$C_6$-Alkenyl, $C_2$-$C_6$-Alkinyl oder $C_1$-$C_6$-Alkoxy steht;
sowie die Salze dieser Verbindungen.

2. Verbindungen der Formel I nach Anspruch 1, worin X für Sauerstoff oder Schwefel steht; $R_1$ für Wasserstoff oder $C_1$-$C_6$-Alkyl steht; und $R_2$ Wasserstoff, $C_1$-$C_6$-Alkyl, $C_2$-$C_6$-Alkenyl, $C_2$-$C_6$-Alkinyl oder $C_1$-$C_6$-Alkoxy bedeutet.

3. Verbindungen der Formel I nach Anspruch 2, worin X für Sauerstoff oder Schwefel steht; $R_1$ für Wasserstoff oder $C_1$-$C_3$-Alkyl steht; und $R_2$ $C_1$-$C_6$-Alkyl oder $C_1$-$C_6$-Alkoxy bedeutet.

4. Verbindungen der Formel I nach Anspruch 3, worin X für Sauerstoff steht; $R_1$ für Wasserstoff oder $C_1$-$C_3$-Alkyl steht; und $R_2$ $C_1$-$C_6$-Alkyl bedeutet.

5. Verbindungen der Formel I nach Anspruch 1, worin X für Sauerstoff oder Schwefel steht; $R_1$ für Wasserstoff oder $C_1$-$C_3$-Alkyl steht; und $R_2$ $C_2$-$C_6$-Alkenyl oder $C_2$-$C_6$-Alkinyl bedeutet.

6. Verbindungen der Formel I nach Anspruch 5, worin X für Sauerstoff steht; $R_1$ für Wasserstoff oder $C_1$-$C_3$-Alkyl steht; und $R_2$ $C_2$-$C_4$-Alkenyl oder $C_2$-$C_4$-Alkinyl bedeutet.

7. Verbindungen der Formel I nach Anspruch 1, worin X für Sauerstoff steht und $R_1$ und $R_2$ unabhängig voneinander für Wasserstoff, $C_2$-$C_6$-Alkenyl oder $C_2$-$C_6$-Alkinyl bedeuten.

8. Eine Verbindung der Formel I nach Anspruch 1, ausgewählt aus der Reihe:
N-[2-(1,2-Dichlorvinyloxy)phenylsulfonyl]-harnstoff (Verb. Nr. 1.1);
N-[2-(1,2-Dichlorvinyloxy)phenylsulfonyl]-N'-methylharnstoff (Verb. Nr. 1.2);
N-[2-(1,2-Dichlorvinyloxy)phenylsulfonyl]-N'-isopropylharnstoff (Verb. Nr. 1.5);
N-[2-(1,2-Dichlorvinyloxy)phenylsulfonyl]-N'-n-butylharnstoff (Verb. Nr. 1.6);
N-[2-(1,2-Dichlorvinyloxy)phenylsulfonyl]-N'-methoxy-N'-methylharnstoff (Verb. Nr. 1.29);
N-[2-(1,2-Dichlorvinyloxy)phenylsulfonyl]-N'-allylharnstoff (Verb. Nr. 1.20);
N-[2-(1,2-Dichlorvinyloxy)phenylsulfonyl]-N',N'-dimethylharnstoff (Verb. Nr. 1.24);
N-[2-(1,2-Dichlorvinyloxy)phenylsulfonyl]-N'-methyl-N'-ethylharnstoff (Verb. Nr. 1.25);
N-[2-(1,2-Dichlorvinyloxy)phenylsulfonyl]-N'-methyl-N'-allylharnstoff (Verb. Nr. 1.32);
N-[2-(1,2-Dichlorvinyloxy)phenylsulfonyl]-N'-methylthioharnstoff (Verb. Nr. 2.2);
N-[2-(1,2-Dichlorvinyloxy)phenylsulfonyl]-N'-isopropylthioharnstoff (Verb. Nr. 2.5);
N-[2-(1,2-Dichlorvinyloxy)phenylsulfonyl]-N'-allylthioharnstoff Verb.Nr. 2.20).

9. Verfahren zur Herstellung von Verbindungen der Formel I

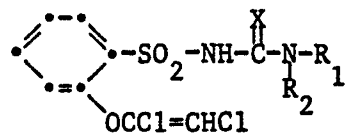

(I)

worin

X für Sauerstoff oder Schwefel steht;

$R_1$ Wasserstoff $C_1$-$C_{12}$-Alkyl, $C_2$-$C_6$-Alkenyl oder $C_2$-$C_6$-Alkinyl bedeutet; und

$R_2$ für Wasserstoff, $C_1$-$C_{12}$-Alkyl, $C_2$-$C_6$-Alkenyl, $C_2$-$C_6$-Alkinyl oder $C_1$-$C_6$-Alkoxy steht; sowie die Salze dieser Verbindungen, dadurch gekennzeichnet, dass man in einem reaktionsinerten, organischen Lösungsmittel bei Temperaturen zwischen -20° und +120° C

a) ein 2-(1,2-Dichlorvinyloxy)phenylsulfonyliso(thio)cyanat der Formel II

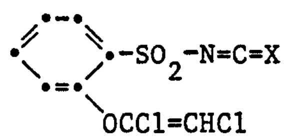

(II)

mit einem Amin der Formel III

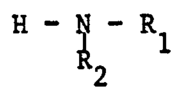

(III)

umsetzt, oder

b) das 2-(1,3-Dichlorvinyloxy)phenylsulfonamid der Formel IV

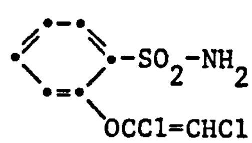

(IV)

in Gegenwart einer Base, mit einem Carbamoylhalogenid der Formel V

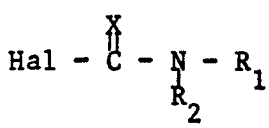

(V)

umsetzt; oder

c) zur Herstellung von Verbindungen der Formel I, worin $R_1$ für Wasserstoff steht, indem man das 2-(1,2-Dichlorvinyloxy)phenylsulfonamid der Formel IV, in Gegenwart einer Base, mit einem Iso(thio)cyanat der Formel VI

$X = C = N - R_2$ (VI)

umsetzt; wobei die Substituenten $R_1$, $R_2$ und X die unter Formel I angegebenen Bedeutungen haben und Hal für Halogen steht.

10. Bakterizides Mittel, dadurch gekennzeichnet, dass es neben landwirtschaftlich geeigneten Trägermaterialien und Formulierungshilfsstoffen als mindestens eine aktive Komponente eine Verbindung der Formel I

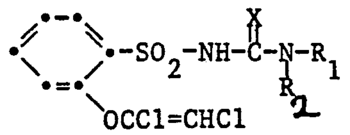

(I),

worin
X für Sauerstoff oder Schwefel steht;
$R_1$ Wasserstoff, $C_1$-$C_{12}$-Alkyl, $C_2$-$C_6$-Alkenyl oder $C_2$-$C_6$-Alkinyl bedeutet; und
$R_2$ für Wasserstoff, $C_1$-$C_{12}$-Alkyl, $C_2$-$C_6$-Alkenyl, $C_2$-$C_6$-Alkinyl oder $C_1$-$C_6$-Alkoxy steht;
oder ein Salz dieser Verbindungen, enthält.

11. Verfahren zur Bekämpfung von phytopathogenen Bakterien, dadurch gekennzeichnet, dass man eine bakterizid wirksame Menge einer Verbindung der Formel I

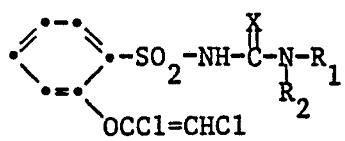

(I),

worin
X für Sauerstoff oder Schwefel steht;
$R_1$ Wasserstoff, $C_1$-$C_{12}$-Alkyl, $C_2$-$C_6$-Alkenyl oder $C_2$-$C_6$-Alkinyl bedeutet; und
$R_2$ für Wasserstoff, $C_1$-$C_{12}$-Alkyl, $C_2$-$C_6$ -Alkenyl, $C_2$-$C_6$-Alkinyl oder $C_1$-$C_6$-Alkoxy steht;
oder ein Salz dieser Verbindungen, auf die Pflanzen oder deren Standort appliziert.

**Patentansprüche**

für den Vertragsstaat AT.

1. Bakterizides Mittel, dadurch gekennzeichnet, dass es neben landwirtschaftlich geeigneten Trägermaterialien und Formulierungshilfsstoffen als mindestens eine aktive Komponente eine Verbindung der Formel I

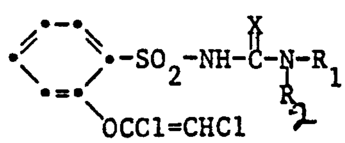

(I)

worin
X für Sauerstoff oder Schwefel steht;
$R_1$ Wasserstoff, $C_1$-$C_{12}$-Alkyl, $C_2$-$C_6$-Alkenyl oder $C_2$ -$C_6$-Alkinyl bedeutet; und
$R_2$ für Wasserstoff, $C_1$-$C_{12}$-Alkyl, $C_2$-$C_6$-Alkenyl, $C_2$-$C_6$-Alkinyl oder $C_1$-$C_6$-Alkoxy steht;
oder ein Salz dieser Verbindungen, enthält.

2. Mittel nach Anspruch 1, enthaltend als Wirkstoff eine Verbindung der Formel I, worin X für Sauerstoff oder Schwefel steht; $R_1$ für Wasserstoff oder $C_1$-$C_6$-Alkyl steht; und $R_2$ Wasserstoff, $C_1$-$C_6$-Alkyl, $C_2$-$C_6$-Alkenyl, $C_2$-$C_6$-Alkinyl oder $C_1$-$C_6$-Alkoxy bedeutet.

3. Mittel nach Anspruch 2, enthaltend als Wirkstoff eine Verbindung der Formel I, worin X für Sauerstoff oder Schwefel steht; $R_1$ für Wasserstoff oder $C_1$-$C_3$-Alkyl steht; und $R_2$ $C_1$-$C_6$-Alkyl oder $C_1$-$C_6$-Alkoxy bedeutet.

4. Mittel nach Anspruch 3, enthaltend als Wirkstoff eine Verbindung der Formel 1, worin X für Sauerstoff steht; $R_1$ für Wasserstoff oder $C_1$-$C_3$-Alkyl steht; und $R_2$ $C_1$-$C_6$-Alkyl bedeutet.

5. Mittel nach Anspruch 1, enthaltend als Wirkstoff eine Verbindung der Formel I, worin X für Sauerstoff oder Schwefel steht; $R_1$ für Wasserstoff oder $C_1$-$C_3$-Alkyl steht; und $R_2$ $C_2$-$C_6$-Alkenyl oder $C_2$-$C_6$-Alkinyl bedeutet.

6. Mittel nach Anspruch 5, enthaltend als Wirkstoff eine Verbindung der Formel I, worin X für Sauerstoff steht; $R_1$ für Wasserstoff oder $C_1$-$C_3$-Alkyl steht; und $R_2$ $C_2$-$C_4$-Alkenyl oder $C_2$-$C_4$-Alkinyl bedeutet.

7. Mittel nach Anspruch 1, enthaltend als Wirkstoff eine Verbindung der Formel I, worin X für Sauerstoff steht und $R_1$ und $R_2$ unabhängig voneinander für Wasserstoff, $C_2$-$C_6$-Alkenyl oder $C_2$-$C_6$ -Alkinyl bedeuten.

8. Mittel nach Anspruch 1, enthaltend als Wirkstoff eine Verbindung der Formel I, ausgewählt aus der Reihe:
N-[2-(1,2-Dichlorvinyloxy)phenylsulfonyl]-harnstoff (Verb. Nr. 1.1);
N-[2-(1,2-Dichlorvinyloxy)phenylsulfonyl]-N'-methylharnstoff (Verb. Nr. 1.2);
N-[2-(1,2-Dichlorvinyloxy)phenylsulfonyl]-N'-isopropylharnstoff (Verb. Nr. 1.5);
N-[2-(1,2-Dichlorvinyloxy)phenylsulfonyl]-N'-n-butylharnstoff (Verb. Nr. 1.6);
N-[2-(1,2-Dichlorvinyloxy)phenylsulfonyl]-N'-methoxy-N'-methyl-harnstoff (Verb. Nr. 1.29);
N-[2-(1,2-Dichlorvinyloxy)phenylsulfonyl]-N'-allylharnstoff (Verb. Nr. 1.20);
N-[2-(1,2-Dichlorvinyloxy)phenylsulfonyl]-N',N'-dimethylharnstoff (Verb. Nr. 1.24);
N-[2-(1,2-Dichlorvinyloxy)phenylsulfonyl]-N'-methyl-N'-ethylharnstoff (Verb. Nr. 1.25);
N-[2-(1,2-Dichlorvinyloxy)phenylsulfonyl]-N'-methyl-N'-allylharnstoff (Verb. Nr. 1.32);
N-[2-(1,2-Dichlorvinyloxy)phenylsulfonyl]-N'-methylthioharnstoff (Verb. Nr. 2.2);
N-[2-(1,2-Dichlorvinyloxy)phenylsulfonyl]-N'-isopropylthioharnstoff (Verb. Nr. 2.5);
N-[2-(1,2-Dichlorvinyloxy)phenylsulfonyl]-N'-allylthioharnstoff Verb.Nr. 2.20).

9. Verfahren zur Herstellung von Verbindungen der Formel I

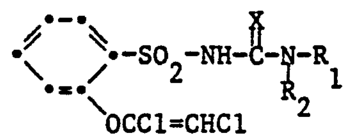 (I)

worin
X für Sauerstoff oder Schwefel steht;
$R_1$ Wasserstoff, $C_1$-$C_{12}$-Alkyl, $C_2$-$C_6$-Alkenyl oder $C_2$-$C_6$-Alkinyl bedeutet; und
$R_2$ für Wasserstoff $C_1$-$C_{12}$-Alkyl, $C_2$-$C_6$-Alkenyl, $C_2$-$C_6$-Alkinyl oder $C_1$-$C_6$-Alkoxy steht; sowie die Salze dieser Verbindungen, dadurch gekennzeichnet, dass man in einem reaktionsinerten, organischen Lösungsmittel bei Temperaturen zwischen -20° und +120°C

a) ein 2-(1,2-Dichlorvinyloxy)phenylsulfonyliso(thio)cyanat der Formel II

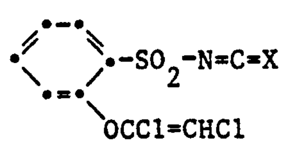 (II)

mit einem Amin der Formel III

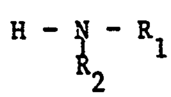 (III)

umsetzt, oder
b) das 2-(1,3-Dichlorvinyloxy)phenylsulfonamid der Formel IV

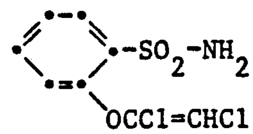

(IV)

in Gegenwart einer Base, mit einem Carbamoylhalogenid der Formel V

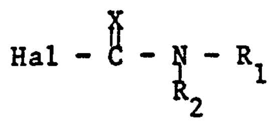

(V)

umsetzt; oder
c) zur Herstellung von Verbindungen der Formel I, worin $R_1$ für Wasserstoff steht, indem man das 2-(1,2-Dichlorvinyloxy)phenylsulfonamid der Formel IV, in Gegenwart einer Base, mit einem Iso(thio)cyanat der Formel VI
$X = C = N - R_2$ (VI)
umsetzt; wobei die Substituenten $R_1$, $R_2$ und X die unter Formel I angegebenen Bedeutungen haben und Hal für Halogen steht.

10. Verfahren zur Bekämpfung von phytopathogenen Bakterien, dadurch gekennzeichnet, dass man eine bakterizid wirksame Menge einer Verbindung der Formel I

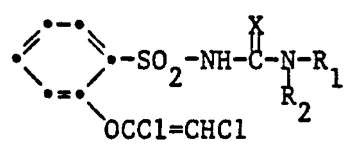

(I)

worin
X für Sauerstoff oder Schwefel steht;
$R_1$ Wasserstoff, $C_1$-$C_{12}$-Alkyl, $C_2$-$C_6$-Alkenyl oder $C_2$-$C_6$-Alkinyl bedeutet; und
$R_2$ für Wasserstoff, $C_1$-$C_{12}$-Alkyl, $C_2$-$C_6$-Alkenyl, $C_2$-$C_6$-Alkinyl oder $C_1$-$C_6$-Alkoxy steht;
oder ein Salz dieser Verbindungen auf die Pflanzen oder deren Standort appliziert.

**Claims**

for the Contracting States: DE, FR, CH, LI, IT, NL, BE, SE, LU.

1. A compound of the formula

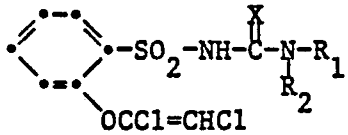

(I)

in which X is oxygen or sulfur; $R_1$ is hydrogen, $C_1$-$C_{12}$-alkyl, $C_2$-$C_6$-alkenyl or $C_2$-$C_6$-alkynyl; and $R_2$ is hydrogen, $C_1$-$C_{12}$-alkyl, $C_2$-$C_6$-alkenyl, $C_2$-$C_6$-alkynyl or $C_1$-$C_6$-alkoxy; or a salt thereof.

2. A compound of the formula I according to claim 1, in which X is oxygen or sulfur, $R_1$ is hydrogen or $C_1$-$C_6$-alkyl; and $R_2$ is hydrogen, $C_1$-$C_6$-alkyl, $C_2$-$C_6$-alkenyl, $C_2$-$C_6$-alkynyl or $C_1$-$C_6$-alkoxy.

3. A compound of the formula I according to claim 2, in which X is oxygen or sulfur; $R_1$ is hydrogen or $C_1$-$C_3$-alkyl; and $R_2$ is $C_1$-$C_6$-alkyl or $C_1$-$C_6$-alkoxy.

4. A compound of the formula I according to claim 3, in which X is oxygen; $R_1$ is hydrogen or $C_1$-$C_3$-alkyl; and $R_2$ is $C_1$-$C_6$-alkyl.

5. A compound of the formula I according to claim 1, in which X is oxygen or sulfur; $R_1$ is hydrogen or $C_1$-$C_3$-alkyl; and $R_2$ is $C_2$-$C_6$-alkenyl or $C_2$-$C_6$-alkynyl.

6. A compound of the formula I according to claim 5, in which X is oxygen; $R_1$ is hydrogen or $C_1$-$C_3$-alkyl; and $R_2$ is $C_2$-$C_4$-alkenyl or $C_2$-$C_4$-alkynyl.

7. A compound of the formula I according to claim 1, in which X is oxygen and each of $R_1$ and $R_2$ independently of the other is hydrogen, $C_2$-$C_6$-alkenyl or $C_2$-$C_6$-alkynyl.

8. A compound of the formula I according to claim 1, selected from the series:
N-[2-(1,2-dichlorovinyloxy)phenylsulfonyl]urea (compound No. 1.1);
N-[2-(1,2-dichlorovinyloxy)phenylsulfonyl]-N'-methylurea (compound No. 1.2);
N-[2-(1,2-dichlorivinyloxy)phenylsulfonyl]-N'-isopropylurea (compound No. 1.5);
N-[2-(1,2-dichlorovinyloxy)phenylsulfonyl]-N'-n-butylurea (compound No. 1.6);
N-[2-(1,2-dichlorovinyloxy)phenylsulfonyl]-N'-methoxy-N'-methylurea (compound No. 1.29);
N-[2-(1,2-dichlorovinyloxy)phenylsulfonyl]-N'-allylurea (compound No. 1.20);
N-[2-(1,2-dichlorovinyloxy)phenylsulfonyl]-N',N'-dimethylurea (compound No. 1.24);
N-[2-(1,2-dichlorovinyloxy)phenylsulfonyl]-N'-methyl-N'-ethylurea (compound No. 1.25);
N-[2-(1,2-dichlorovinyloxy)phenylsulfonyl]-N'-methyl-N'-allylurea (compound No. 1.32);
N-[2-(1,2-dichlorovinyloxy)phenylsulfonyl]-N'-methylthiourea (compound No. 2.2);
N-[2-(1,2-dichlorovinyloxy)phenylsulfonyl]-N'-isopropylthiourea (compound No. 2.5) and
N-[2-(1,2-dichlorovinyloxy)phenylsulfonyl]-N'-allylthiourea (compound No. 2.20).

9. A process for the preparation of a compound of the formula I

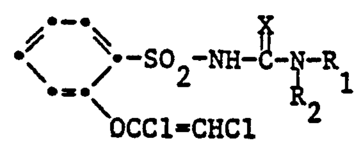

(I)

in which X is oxygen or sulfur; $R_1$ is hydrogen, $C_1$-$C_{12}$-alkyl, $C_2$-$C_6$-alkenyl or $C_2$-$C_6$-alkynyl; and $R_2$ is hydrogen, $C_1$-$C_{12}$-alkyl, $C_2$-$C_6$-alkenyl, $C_2$-$C_6$-alkynyl or $C_1$-$C_6$-alkoxy; or of a salt thereof, which process comprises, in an inert organic solvent and in the temperature range from -20° to +120°C,

a) reacting a 2-(1,2-dichlorovinyloxy)phenylsulfonyl iso(thio)cyanate of the formula II

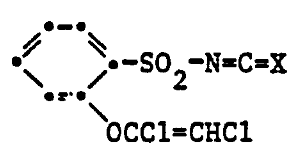

(II)

with an amine of the formula III

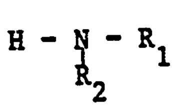

(III)

or

b) reacting 2-(1,2-dichlorovinyloxy)phenylsulfonamide of the formula IV

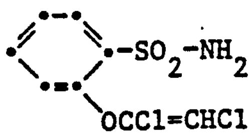

(IV)

with a carbamyl halide of the formula V

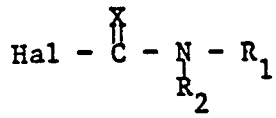

(V)

in the presence of a base, or

c) for the preparation of a compound of the formula I in which $R_1$ is hydrogen, reacting 2-(1,2-dichlorovinyloxy)phenylsulfonamide of the above formula IV with an iso(thio)cyanate of the formula VI

X = C = N - $R_2$ (VI)

in the presence of a base; in which formulae the substituents $R_1$, $R_2$ and X are as defined for formula I and Hal is halogen.

10. A bactericidal composition which contains, as active ingredient, at least one compound of the formula I

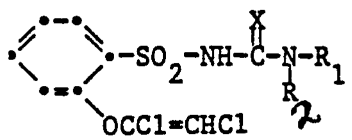

(I)

in which X is oxygen or sulfur; $R_1$ is hydrogen, $C_1$-$C_{12}$-alkyl, $C_2$-$C_6$-alkenyl or $C_2$-$C_6$-alkynyl; and $R_2$ is hydrogen, $C_1$-$C_{12}$-alkyl, $C_2$-$C_6$-alkenyl, $C_2$-$C_6$-alkynyl or $C_1$-$C_6$-alkoxy, or a salt thereof, together with agriculturally acceptable carriers and formulation assistants.

11. A method of combating phytopathogenic bacteria, which method comprises applying to the plants or to the locus thereof a bactericidally effective amount of a compound of the formula I

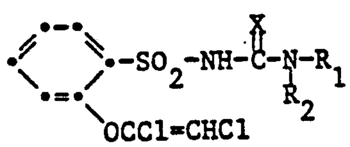

(I),

in which X is oxygen or sulfur, $R_1$ is hydrogen, $C_1$-$C_{12}$-alkyl, $C_2$-$C_6$-alkenyl or $C_2$-$C_6$-alkinyl; and $R_2$ is hydrogen, $C_1$-$C_{12}$-alkyl, $C_2$-$C_6$-alkenyl, $C_2$-$C_6$-alkinyl or $C_1$-$C_6$-alkoxy; or of a salt thereof.

**Claims**

for the Contracting State: AT.

1. A bactericidal composition which contains, as active ingredient, at least one compound of the formula I

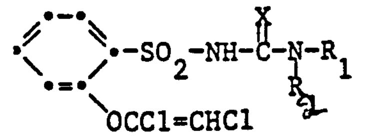

(I)

in which X is oxygen or sulfur; $R_1$ is hydrogen, $C_1$-$C_{12}$-alkyl, $C_2$-$C_6$-alkenyl or $C_2$-$C_6$-alkynyl; and $R_2$ is hydrogen, $C_1$-$C_{12}$-alkyl, $C_2$-$C_6$-alkenyl, $C_2$-$C_6$-alkynyl or $C_1$-$C_6$-alkoxy, or a salt thereof together with agriculturally acceptable carriers and formulation assistants.

2. A composition according to claim 1, which contains, as active ingredient, a compound of formula I, in which X is oxygen or sulfur, $R_1$ is hydrogen or $C_1$-$C_6$-alkyl; and $R_2$ is hydrogen, $C_1$-$C_6$-alkyl, $C_2$-$C_6$-alkenyl, $C_2$-$C_6$-alkynyl or $C_1$-$C_6$-alkoxy.

3. A composition according to claim 2, which contains, as active ingredient, a compound of formula I, in which X is oxygen or sulfur; $R_1$ is hydrogen or $C_1$-$C_3$-alkyl; and $R_2$ is $C_1$-$C_6$-alkyl or $C_1$-$C_6$-alkoxy.

4. A composition according to claim 3, which contains, as active ingredient, a compound of formula I, in which X is oxygen; $R_1$ is hydrogen or $C_1$-$C_3$-alkyl; and $R_2$ is $C_1$-$C_6$-alkyl.

5. A composition according to claim 1, which contains, as active ingredient, a compound of formula I, in which X is oxygen or sulfur; $R_1$ is hydrogen or $C_1$-$C_3$-alkyl; and $R_2$ is $C_2$-$C_6$-alkenyl or $C_2$-$C_6$-alkynyl.

6. A composition according to claim 5, which contains, as active ingredient, a compound of formula I, in which X is oxygen; $R_1$ is hydrogen or $C_1$-$C_3$-alkyl; and $R_2$ is $C_2$-$C_4$-alkenyl or $C_2$-$C_4$-alkynyl.

7. A composition according to claim 1, which contains, as active ingredient, a compound of formula I, in which X is oxygen and each of $R_1$ and $R_2$ independently of the other is hydrogen, $C_2$-$C_6$-alkenyl or $C_2$-$C_6$-alkynyl.

8. A composition according to claim 1, which contains, as active ingredient, a compound of formula I, selected from the series:

N-[2-(1,2-dichlorovinyloxy)phenylsulfonyl]urea (compound No. 1.1);
N-[2-(1,2-dichlorovinyloxy)phenylsulfonyl]-N'-methylurea (compound No. 1.2);
N-[2-(1,2-dichlorivinyloxy)phenylsulfonyl]-N'-isopropylurea (compound No. 1.5);
N-[2-(1,2-dichlorovinyloxy)phenylsulfonyl]-N'-n-butylurea (compound No. 1.6);
N-[2-(1,2-dichlorovinyloxy)phenylsulfonyl]-N'-methoxy-N'-methylurea (compound No. 1.29);
N-[2-(1,2-dichlorovinyloxy)phenylsulfonyl]-N'-allylurea (compound No. 1.20);
N-[2-(1,2-dichlorovinyloxy)phenylsulfonyl]-N',N'-dimethylurea (compound No. 1.24);
N-[2-(1,2-dichlorovinyloxy)phenylsulfonyl]-N'-methyl-N'-ethylurea (compound No. 1.25);
N-[2-(1,2-dichlorovinyloxy)phenylsulfonyl]-N'-methyl-N'-allylurea (compound No. 1.32);
N-[2-(1,2-dichlorovinyloxy)phenylsulfonyl]-N'-methylthiourea (compound No. 2.2);
N-[2-(1,2-dichlorovinyloxy)phenylsulfonyl]-N'-isopropylthiourea (compound No. 2.5) and
N-[2-(1,2-dichlorovinyloxy)phenylsulfonyl]-N'-allylthiourea (compound No. 2.20).

9. A process for the preparation of a compound of the formula I

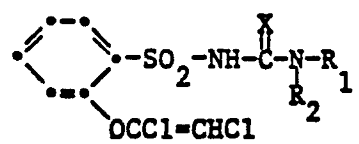

(I)

in which X is oxygen or sulfur; $R_1$ is hydrogen, $C_1$-$C_{12}$-alkyl, $C_2$-$C_6$-alkenyl or $C_2$-$C_6$-alkynyl; and $R_2$ is hydrogen, $C_1$-$C_{12}$-alkyl, $C_2$-$C_6$-alkenyl, $C_2$-$C_6$-alkynyl or $C_1$-$C_6$-alkoxy; or of a salt thereof, which process comprises in an inert oorganic solvent and in the temperature range from -20° to +120° C,

a) reacting a 2-(1,2-dichlorovinyloxy)phenylsulfonyl iso(thio)cyanate of the formula II

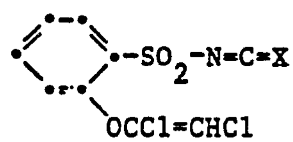

(II)

with an amine of the formula III

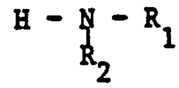 (III)

or

b) reacting 2-(1,2-dichlorovinyloxy)phenylsulfonamide of the formula IV

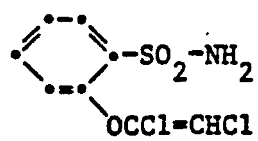 (IV)

with a carbamyl halide of the formula V

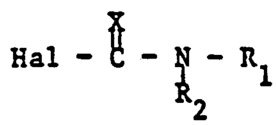 (V)

in the presence of a base, or

c) for the preparation of a compound of the formula I in which $R_1$ is hydrogen, reacting 2-(1,2-dichlorovinyloxy)phenylsulfonamide of the above formula IV with an iso(thio)cyanate of the formula VI

$X = C = N - R_2$ (VI)

in the presence of a base; in which formulae the substituents $R_1$, $R_2$ and X are as defined for formula I and Hal is halogen.

10. A method of combating phytopathogenic bacteria, which method comprises applying to the plants or to the locus thereof a bactericidally effective amount of a compound of the formula I

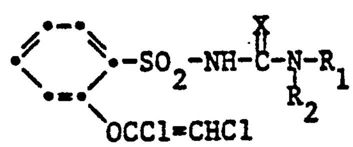 (I),

in which X is oxygen or sulfur, $R_1$ is hydrogen, $C_1$-$C_{12}$-alkyl, $C_2$-$C_6$-alkenyl or $C_2$-$C_6$-alkinyl; and $R_2$ is hydrogen, $C_1$-$C_{12}$-alkyl, $C_2$-$C_6$-alkenyl, $C_2$-$C_6$-alkinyl or $C_1$-$C_6$-alkoxy; or of a salt thereof.

**Revendications**

pour les Etats contractants: BE, CH, LI, DE, FR, IT, LU, NL, SE.

1. Composés de formule

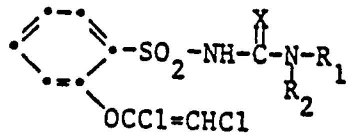

(I)

où

X représente un oxygène ou un soufre;

$R_1$ représente un hydrogène, un alkyle $C_1$-$C_{12,}$ un alcényle $C_2$-$C_6$ ou un alcynyle $C_2$-$C_6$; et

$R_2$ représente un hydrogène, un alkyle $C_1$-$C_{12,}$ un alcényle $C_2$-$C_{6,}$ un alcynyle $C_2$-$C_6$ ou un alkoxy $C_1$-$C_6$;

ainsi que les sels de ces composés.

2. Composés de formule I selon la revendication 1, où X représente un oxygène ou un soufre; $R_1$ représente un hydrogène ou un alkyle $C_1$-$C_6$; et $R_2$ représente un hydrogène, un alkyle $C_1$-$C_{6,}$ un alcényle $C_2$-$C_{6,}$ un alcynyle $C_2$-$C_6$ ou un alkoxy $C_1$ $C_6$.

3. Composés de formule I selon la revendication 2, où X représente un oxygène ou un soufre; $R_1$ représente un hydrogène ou un alkyle $C_1$-$C_3$; et $R_2$ représente un alkyle $C_1$-$C_6$ ou un alkoxy $C_1$ $C_6$.

4. Composés de formule I selon la revendication 3, où X représente un oxygène; $R_1$ représente un hydrogène ou un alkyle $C_1$-$C_3$; et $R_2$ représente un alkyle $C_1$-$C_6$.

5. Composés de formule I selon la revendication 1, où X représente un oxygène ou un soufre; $R_1$ représente un hydrogène ou un alkyle $C_1$-$C_3$; et $R_2$ représente un alcényle $C_2$-$C_6$ ou un alcynyle $C_2$-$C_6$.

6. Composés de formule I selon la revendication 5, où X représente un oxygène; $R_1$ représente un hydrogène ou un alkyle $C_1$-$C_3$; et $R_2$ représente un alcényle $C_2$-$C_4$ ou un alcynyle $C_2$-$C_4$.

7. Composés de formule I selon la revendication 1, où X représente un oxygène et $R_1$ et $R_2$ représentent indépendamment l'un de l'autre un hydrogène, un alcényle $C_2$-$C_6$ ou un alcynyle $C_2$-$C_6$.

8. Un composé de formule I selon la revendication 1, choisi dans la série suivante:

N-[2-(1,2-dichlorovinyloxy)phénylsulfonyl]urée (Comp. n° 1.1);

N-[2-(1,2-dichlorovinyloxy)phénylsulfonyl]-N'-méthylurée (Comp. n° 1.2);

N-[2-(1,2-dichlorovinyloxy)phénylsulfonyl]-N'-iso-propylurée (Comp. n° 1.5);

N-[2-(1,2-dichlorovinyloxy)phénylsulfonyl]-N'-n-butylurée (Comp. n° 1.6);

N-[2-(1,2-dichlorovinyloxy)phénylsulfonyl]-N'-méthoxy-N'-méthylurée (Comp. n° 1.29);

N-[2-(1,2-dichlorovinyloxy)phénylsulfonyl]-N'-allylurée (Comp. n° 1.20);

N-[2-(1,2-dichlorovinyloxy)phénylsulfonyl]-N',N'-diméthylurée (Comp. n° 1.24);

N-[2-(1,2-dichlorovinyloxy)phénylsulfonyl)-N'-méthyl-N'-éthylurée (Comp. n° 1.25);

N-[2-(1,2-dichlorovinyloxy)phénylsulfonyl]-N'-méthyl-N'-allylurée (Comp. n° 1.32);

N-[2-(1,2-dichlorovinyloxy)phénylsulfonyl]-N'-méthyl-thiourée (Comp. n° 2.2);

N-[2-(7,2-dichlorovinyloxy)phénylsulfonyl]N'-iso-propylthio-urée (Comp. n° 2.5);

N-[2-(1,2-dichlorovinyloxy)phénylsulfonyl]-N'-allyl-thio-urée (Comp. n° 2.20).

9. Procédé de préparation de composés de formule I

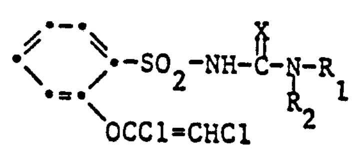

(I)

où

X représente un oxygène ou un soufre;

$R_1$ représente un hydrogène, un alkyle $C_1$-$C_{12,}$ un alcényle $C_2$-$C_6$ ou un alcynyle $C_2$-$C_6$; et

$R_2$ représente un hydrogène, un alkyle $C_1$-$C_{12,}$ un alcényle $C_2$-$C_{6,}$ un alcynyle $C_2$-$C_6$ ou un alkoxy $C_1$-$C_6$; ainsi que les sels de ces composés, caractérisé en ce que, dans un solvant organique inerte, à des températures comprises entre -20° et +120°C,

a) on fait réagir un 2-(1,2-dichlorovinyloxy)phényl-sulfonyliso(thio)cyanate de formule II

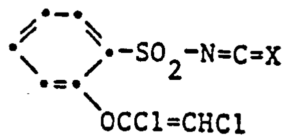

(II)

avec une amine de formule III

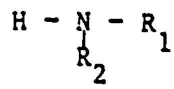

(III)

ou

b) on fait réagir le 2-(1,2-dichlorovinyloxy)phényl-sulfamide de formule IV

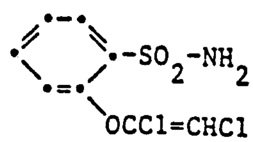

(IV)

en présence d'une base avec un halogénure de carbamoyle de formule V

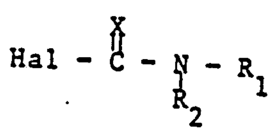

(V)

ou

c) pour la préparation de composés de formule I, où $R_1$ représente un hydrogène, on fait réagir le 2-(1,2-dichlorovinyloxy)phénylsulfamide de formule IV, en présence d'une base, avec un iso(thio)cyanate de formule VI

$X = C = N - R_2$ (VI)

les substituants $R_1$, $R_2$ et X ayant les significations indiquées dans la formule I et Hal représente un halogène.

10. Produit bactéricide caractérisé en ce que, à côté d'excipients appropriés sur le plan de l'agriculture et d'adjuvants de composition, il contient au moins en tant qu'un constituant actif un composé de formule I

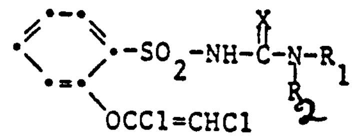 (I),

où
X représente un oxygène ou un soufre;
$R_1$ représente un hydrogène, un alkyle $C_1$-$C_{12}$, un alcényle $C_2$-$C_6$ ou un alcynyle $C_2$-$C_6$; et
$R_2$ représente un hydrogène, un alkyle $C_1$-$C_{12}$, un alcényle $C_2$-$C_6$, un alcynyle $C_2$-$C_6$ ou un alkoxy $C_1$-$C_6$;
ou un sel de ces composés.

11. Procédé de lutte contre les bactéries phyto-pathogènes, caractérisé en ce qu'on applique aux plantes ou à leur habitat une quantité bactéricide efficace d'un composé de formule I

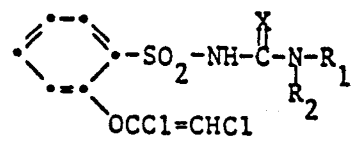 (I)

où
X représente un oxygène ou un soufre;
$R_1$ représente un hydrogène, un alkyle $C_1$-$C_{12}$, un alcényle $C_2$-$C_6$ ou un alcynyle $C_2$-$C_6$; et
$R_2$ représente un hydrogène, un alkyle $C_1$-$C_{12}$, un alcényle $C_2$-$C_6$, un alcynyle $C_2$-$C_6$ ou un alkoxy $C_1$-$C_6$;
ou un sel de ces composés.

**Revendications**

pour l'état contractant: AT.

1. Produit bactéricide caractérisé en ce que, à côté d'excipients appropriés sur le plan de l'agriculture et d'adjuvants de composition, il contient au moins en tant qu'un constituant actif un composé de formule I

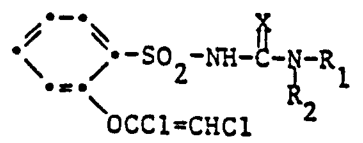 (I)

où
X représente un oxygène ou un soufre;
$R_1$ représente un hydrogène, un alkyle $C_1$-$C_{12}$, un alcényle $C_2$-$C_6$ ou un alcynyle $C_2$-$C_6$; et
$R_2$ représente un hydrogène, un alkyle $C_1$-$C_{12}$, un alcényle $C_2$-$C_6$, un alcynyle $C_2$-$C_6$ ou un alkoxy $C_1$-$C_6$;
ou un sel de ces composés.

2. Produit selon la revendication 1, contenant en tant que principe actif un composé de formule I, où X représente un oxygène ou un soufre; $R_1$ représente un hydrogène ou un alkyle $C_1$-$C_6$; et $R_2$ représente un hydrogène, un alkyle $C_1$-$C_6$, un alcényle $C_2$-$C_6$, un alcynyle $C_2$-$C_6$ ou un alkoxy $C_1$-$C_6$.

3. Produit selon la revendication 2, contenant en tant que principe actif un composé de formule I, où X représente un oxygène ou un soufre; $R_1$ représente un hydrogène ou un alkyle $C_1$-$C_3$; et $R_2$ représente un alkyle $C_1$-$C_6$ ou un alkoxy $C_1$-$C_6$.

4. Produit selon la revendication 3, contenant en tant que principe actif un composé de formule I, où X représente un oxygène; $R_1$ représente un hydrogène ou un alkyle $C_1$-$C_3$; et $R_2$ représente un alkyle $C_1$-$C_6$.

5. Produit selon la revendication 1, contenant en tant que principe actif un composé de formule I, où X représente un oxygène ou un soufre; $R_1$ représente un hydrogène ou un alkyle $C_1$-$C_3$; et $R_2$ représente un alcényle $C_2$-$C_6$ ou un alcynyle $C_2$-$C_6$.

6. Produit selon la revendication 5, contenant en tant que principe actif un composé de formule I, où X représente un oxygène; $R_1$ représente un hydrogène ou un alkyle $C_1$-$C_3$; et $R_2$ représente un alcényle $C_2$-$C_4$ ou un alcynyle $C_2$-$C_4$.

7. Produit selon la revendication I, contenant en tant que principe actif un composé de formule I, où X représente un oxygène et $R_1$ et $R_2$ représentent indépendamment l'un de l'autre un hydrogène, un alcényle $C_2$-$C_6$ ou un alcynyle $C_2$-$C_6$.

8. Produit selon la revendication 1, contenant en tant que principe actif un composé de formule I, choisi dans la série suivante:

N-[2-(1,2-dichlorovinyloxy)phénylsulfonyl]-urée (Comp. n° 1.1);
N-[2-(1,2-dichlorovinyloxy)phénylsulfonyl]-N'-méthylurée (Com. n° 1.2);
N-[2-(1,2-dichlorovinyloxy)phénylsulfonyl]-N'-iso-propylurée (Comp. n° 1.5);
N-[2-(1,2-dichlorovinyloxy)phénylsulfonyl]-N'-n-butylurée (Comp. n° 1.6);
N-[2-(1,2-dichlorovinyloxy)phénylsulfonyl]-N'-méthoxy-N'-méthylurée (Comp. n° 1.29);
N-[2-(1,2-dichlorovinyloxy)phénylsulfonyl]-N'-allylurée (Comp. n° 1.20);
N-[2-(1,2-dichlorovinyloxy)phénylsulfonyl]-N',N'-diméthylurée (Comp. n° 1.24);
N-[2-(1,2-dichlorovinyloxy)phénylsulfonyl]-N'-méthyl-N'-éthylurée (Comp. n° 1.25);
N-(2-(1,2-dichlorovinyloxy)phénylsulfonyl)-N'-méthyl-N'-allylurée (Comp. n° 1.32);
N-[2-(1,2-dichlorovinyloxy)phénylsulfonyl)-N'-méthyl-thiourée (Comp. n° 2.2);
N-[2-(1,2-dichlorovinyloxy)phénylsulfonyl]-N'-iso-propylthio-urée (Comp. n° 2.5);
N-[2-(1,2-dichlorovinyloxy)phénylsulfonyl]-N'-allyl-thio-urée (Comp. n° 2.20).

9. Procédé de préparation de composés de formule I

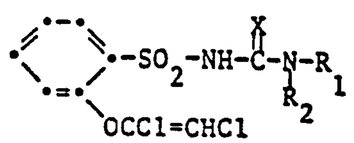

(I)

où

X représente un oxygène ou un soufre;

$R_1$ représente un hydrogène, un alkyle $C_1$-$C_{12}$, un alcényle $C_2$-$C_6$ ou un alcynyle $C_2$-$C_6$; et

$R_2$ représente un hydrogène, un alkyle $C_1$-$C_{12}$, un alcényle $C_2$-$C_6$, un alcynyle $C_2$-$C_6$ ou un alkoxy $C_1$-$C_6$ ainsi que les sels de ces composés, caractérisé en ce que, dans un solvant organique inerte, à des températures comprises entre -20° et +120°C,

a) on fait réagir un 2-(1,2-dichlorovinyloxy)phényl-sulfonyliso(thio)cyanate de formule II

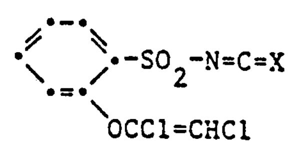

(II)

avec une amine de formule III

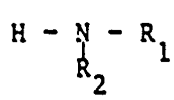

(III)

ou

b) on fait réagir le 2-(1,2-dichlorovinyloxy)phényl-sulfamide de formule IV

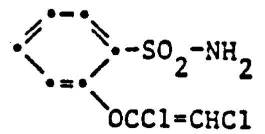

(IV)

en présence d'une base avec un halogénure de carbamoyle de formule V

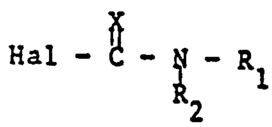

(V)

ou

c) pour la préparation de composés de formule I, où $R_1$ représente un hydrogène, on fait réagir le 2-(1,2-dichlorovinyloxy)phénylsulfamide de formule IV, en présence d'une base, avec un iso(thio)cyanate de formule VI

$X = C = N - R_2$ (VI)

les substituants $R_1$, $R_2$ et X ayant les significations indiquées dans la formule I et Hal représente un halogène.

10. Procédé de lutte contre les bactéries phytopathogènes, caractérisé en ce qu'on applique aux plantes ou à leur habitat une quantité bactéricide d'un composé de formule I

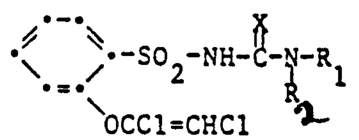

(I),

où

X représente un oxygène ou un soufre;

$R_1$ représente un hydrogène, un alkyle $C_1$-$C_{12}$, un alcényle $C_2$-$C_6$ ou un alcynyle $C_2$-$C_6$; et

$R_2$ représente un hydrogène, un alkyle $C_1$-$C_{12}$, un alcényle $C_2$-$C_6$, un alcynyle $C_2$-$C_6$ ou un alkoxy $C_1$-$C_6$ ou un sel de ces composés.